# EUROPEAN PATENT SPECIFICATION

(11) **EP 3 972 581 B1**
(45) Date of publication and mention of the grant of the patent: **25.03.2026**
(21) Application number: 20725561.3
(22) Date of filing: 18.05.2020
(51) Int. Cl.: A61K 31/202, A61K 31/732, A61K 31/7004, A61K 45/06, A61P 1/16, A61P 3/04, A61P 3/06, A61P 3/10, A61P 9/00, A61K 9/48, A23L 33/12, A23L 33/125, A23L 33/175, A61K 35/00, A61K 38/00

(54) **PREBIOTIC COMPOSITIONS**
PRÄBIOTISCHE ZUSAMMENSETZUNGEN
COMPOSITIONS PRÉBIOTIQUES

(30) Priority: 23.05.2019 EP 19176071
(43) Date of publication of application: 30.03.2022
(73) Proprietor: Evonik Operations GmbH, 45128 Essen (DE)
(72) Inventor: SPECKMANN, Bodo, 63796 Kahl (DE); STUDTE, Christopher, 64293 Darmstadt (DE)
(74) Representative: Evonik Patent Association
(86) International application number: PCT/EP2020/063775
(87) International publication number: WO 2020/234221

(56) References cited:
- WO-A1-2019/008101
- DE-A1- 10 214 005
- MASANORI KATAKURA ET AL: "Omega-3 Fatty Acids Protect Renal Functions by Increasing Docosahexaenoic Acid-Derived Metabolite Levels in SHR.Cg-Leprcp/NDmcr Rats, a Metabolic Syndrome Model", MOLECULES, vol. 19, no. 3, 17 March 2014 (2014-03-17), pages 3247 - 3263, XP055670184, DOI: 10.3390/molecules19033247
- LARA COSTANTINI ET AL: "Impact of Omega-3 Fatty Acids on the Gut Microbiota", INTERNATIONAL JOURNAL OF MOLECULAR SCIENCES, vol. 18, no. 12, 7 December 2017 (2017-12-07), pages 2645, XP055634451, DOI: 10.3390/ijms18122645
- JANET A VOGT ET AL: "l-Rhamnose increases serum propionate in humans", THE AMERICAN JOURNAL OF CLINICAL NUTRITION, vol. 80, no. 1, 1 July 2004 (2004-07-01), US, pages 89 - 94, XP055642663, ISSN: 0002-9165, DOI: 10.1093/ajcn/80.1.89
- SO-JUNG BANG ET AL: "The influence of in vitro pectin fermentation on the human fecal microbiome", AMB EXPRESS, vol. 8, no. 1, 16 June 2018 (2018-06-16), XP055670382, DOI: 10.1186/s13568-018-0629-9
- SEHAD ALARIFI ET AL: "In vitro fermentation of gum acacia - impact on the faecal microbiota", INTERNATIONAL JOURNAL OF FOOD SCIENCES AND NUTRITION, vol. 69, no. 6, 16 January 2018 (2018-01-16), GB, pages 696 - 704, XP055670379, ISSN: 0963-7486, DOI: 10.1080/09637486.2017.1404970
- EVELIEN VAN RYMENANT ET AL: "A Critical Evaluation of In Vitro Hesperidin 2S Bioavailability in a Model Combining Luminal (Microbial) Digestion and Caco-2 Cell Absorption in Comparison to a Randomized Controlled Human Trial", MOLECULAR NUTRITION & FOOD RESEARCH, vol. 62, no. 8, 1 April 2018 (2018-04-01), DE, pages 1700881, XP055670388, ISSN: 1613-4125, DOI: 10.1002/mnfr.201700881
- DOUGLAS J. MORRISON ET AL: "Formation of short chain fatty acids by the gut microbiota and their impact on human metabolism", GUT MICROBES, vol. 7, no. 3, 10 March 2016 (2016-03-10), United States, pages 189 - 200, XP055374912, ISSN: 1949-0976, DOI: 10.1080/19490976.2015.1134082
- CAMUESCO ET AL: "Intestinal anti-inflammatory activity of combined quercitrin and dietary olive oil supplemented with fish oil, rich in EPA and DHA (n-3) polyunsaturated fatty acids, in rats with DSS-induced colitis", CLINICAL NUTRITION, CHURCHILL LIVINGSTONE, LONDON, GB, vol. 25, no. 3, 1 June 2006 (2006-06-01), pages 466 - 476, XP005492242, ISSN: 0261-5614, DOI: 10.1016/J.CLNU.2005.12.009

## Description

This invention concerns preparations comprising L-rhamnose, and the omega-3 fatty acids eicosapentaenoic acid (EPA) and docosahexaenoic acid (DHA) in the form of either fatty acid salts having an organic counter ion selected from lysine, arginine, ornithine, choline and mixtures of the same, or mixtures of free fatty acids and omega-3 fatty acid salts having an organic counter ion selected from lysine, arginine, ornithine, choline and a preparation for use as a feed or food

supplement as specified in the appended claims. Type 2 diabetes (T2D) and cardiovascular diseases (CVD) are among the leading causes of morbidity and deaths worldwide. Both diseases often arise subsequent to a manifest metabolic syndrome (MetS). MetS, T2D, and CVD not only impair the quality of life of affected persons but also challenge public health care systems. Consequently, there is an immense demand for novel strategies to prevent, ameliorate and cure these diseases/conditions. The gastrointestinal microbiota modulates health and has therefore emerged as a target of interventions to improve the health of humans and animals. Microbiota-targeted strategies include the application of prebiotics, probiotics and synbiotics to modulate the microbiota's composition and activity. Prebiotics support the growth of beneficial microbes; a host-centered definition describes a prebiotic as "a substrate that is selectively utilized by host microorganisms conferring a health benefit" (Consensus definition by the International Scientific Association for Probiotics and Prebiotics (ISAPP)) [1]. The most commonly investigated and applied prebiotics are fructo-oligosaccharides (FOS), galacto-oligosaccharides (GOS), arabinoxylan-oligosaccharides (AXOS), xylo-oligosaccharides (XOS), and beta-glucans. Probiotics are defined as: "live microorganisms that, when administered in adequate amounts, confer a health benefit on the host" (ISAPP definition) [2]. The most commonly investigated and commercially available probiotics are mainly microorganisms from species of genera *Lactobacillus* and *Bifidobacterium.* In addition, several others such as *Propionibacterium, Streptococcus, Bacillus, Enterococcus, Escherichia coli,* and yeasts are also used. In the context of this invention, we understand the term "synbiotics" as combinations of probiotics with any chemically defined substance(/s), e.g. amino acids, peptides, fatty acids, and carbohydrates.

The gut microbiota exerts beneficial effects on the host by e.g. production of substances like phenolic acids, indole derivatives, diacetyl, and short-chain fatty acids (SCFA). Favorable SCFA are in particular acetate, propionate, and butyrate, which act upon the host locally in the gut mucosa and/or systemically after they have reached the portal vein and are subsequently transported to various tissues or organs [3]. The systemic availability of colonic SCFA is highest for acetate (~ 36%), ~ 9% for propionate, and lowest for butyrate (~ 2 %) [4]. Beneficial effects attributed to an increased intestinal SCFA production include the improvement of gut barrier function, anti-inflammatory and immune-modulatory effects, production of satiety-promoting hormones, as well as interactions with glucose and lipid metabolism. These attributions imply that SCFA are a promising target to control in particular metabolic disorders such as type 2 diabetes, traits of the metabolic syndrome, and cardiovascular diseases.

Most of the scientific literature about SCFA focuses on butyrate, while the roles of propionate in physiology and pathology have only more recently begun to be discovered. Plasma concentrations of propionate are inversely correlated with LDL-cholesterol and total cholesterol in healthy males [5]. Causality of this link is inferred by hypocholesterolemic effects of supplemented propionate in rats [6]. Likewise, studies with isolated hepatocytes showed that propionate inhibits cholesterol and fatty acid biosynthesis [7, 8]. Moreover, feeding mice on a high-fat diet with propionate, but not with acetate, significantly attenuated hepatic lipogenesis *in vivo* [9]. In contrast to acetate, which is transformed to acetyl-CoA in the liver, propionate does not contribute to the *de novo* synthesis of even-chain fatty acids (mainly C16:0) or cholesterol, which is of importance for individuals with hyperlipidemia or hypercholesterolemia. Propionate feeds instead into the pool of odd-chain fatty acids (e.g. C15:0 and C17:0) [9, 10], which are linked to improved insulin sensitivity & diabetes prevention and therefore may be relevant mediators of propionate's beneficial health effects. Some of the findings from animal studies have been translated to humans, as exemplified by decreases of hepatic lipid content, insulin resistance, and weight gain of obese subjects receiving 10g inulin-propionate/day for 24 weeks [11]. Such treatment also increased the levels of satiety hormones GLP-1 and PYY and reduced the participants' energy intake.

Propionate occurs naturally in low quantities in milk and in dairy products like yogurt and cheese. Propionic acid (PA) salts are used (in very limited amounts) as food and feed preservatives because of their antifungal and antimicrobial activities. Albeit PA-containing supplements are available, the oral delivery of PA is not favorable because of its rancid odor and because it is a relatively strong acid (pK_{A} = 4.88) that irritates the sensitive mucosal tissues. Moreover, a sudden intraluminal release of preformed PA from a carrier would exacerbate this irritating effect and at the same time compromise possible physiological benefits, which require a more sustained uptake of propionate from the gastrointestinal tract. Colonic delivery of propionate through capsules coated with EUDRAGIT^{®} S100 (Evonik Nutrition and Care GmbH, Darmstadt, Germany) has indeed been shown to result in a rapid and short-term increase of plasma propionate levels [4].

A more promising approach is to promote the endogenous formation of propionate by the application of microbiome modulators, e.g. pre- and probiotics. Colonic fermentation of undigested food is the prime source of luminal and circulating propionate [12, 13]. Some amino acids, e.g. asparagine, are fermented to propionate [14]. Larger amounts of propionate derive from a broad range of prebiotic carbohydrates via three known microbial metabolization routes [4]. Taxa involved in these routes include *Propionibacterium, Prevotella, Veillonella alcalescens, Clostridium propionicum, Selenomonas, Micromonospora, Bacteroides,* and *Ruminococcus.* A screening of relevant prebiotics revealed that L-rhamnose is the most potent substrate for production of propionate by human gut microbes [5]. Human trials assessing circulating propionate levels after L-rhamnose supplementation gave however mixed results; one trial reported a significant increase after acute intake of 25 g L-rhamnose [15], whereas an intake of up to 25,5 g L-rhamnose/day for seven days showed no increase of serum propionate [16]. We reason that an effective acceleration of endogenous propionate production depends on a number of factors, including identity, quantity & source of the prebiotic or -more general- substance, its formulation, transit time, and the microbiota composition. Costantini L. et al., Journal of Molecular Sciences, 2017, vol. 18, no. 12, pg. 2645 discloses the omega-3 fatty acids EPA and DHA to increase short chain fatty acids (SCFA) producing bacteria. Vogt J. et al., American Journal of Clinical Nutrition, 2004, vol. 80, no. 1, pg. 89-94 discloses L-rhamnose to increase serum propionate in humans.

The limitations of currently available intervention strategies show the need for more advanced technologies to improve endogenous propionate levels and subsequent health effects in a more reliable and feasible manner.

Therefore, it was an objective of the present invention to provide new applications of omega-3 fatty acids for promoting production of the favorable SCFA, such as propionate in the gastrointestinal tract. This invention discloses a technology that promotes formation of propionate *in vivo,* i.e. in the large intestine of an animal or human being, by novel compositions of the compounds L-rhamnose, and suitable derivatives of EPA and DHA in a suitable formulation, such that the combination technology delivers enhanced propionate levels and propionate/acetate ratios. An advantage of the technology is that it delivers a high concentration of active ingredients into the colon, where these ingredients serve as substrates for fermentations towards propionate and at the same time modulate the microbiota towards an increase of propionate-producing taxa. Such technology can provide a benefit to humans and animals suffering from the above-mentioned metabolic and chronic inflammatory conditions and that are in need of novel strategies to prevent, ameliorate or cure such and similar conditions. Therefore, the present invention is directed to preparations comprising L-rhamnose, and the omega-3 fatty acids eicosapentaenoic acid (EPA) and docosahexaenoic acid (DHA) in the form of either fatty acid salts having an organic counter ion selected from lysine, arginine, ornithine, choline and mixtures of the same, or mixtures of free fatty acids and omega-3 fatty acid salts having an organic counter ion selected from lysine, arginine, ornithine, choline and a preparation for use as a feed or food supplement as specified in the appended claims.

Surprisingly, it was found that omega-3 fatty acids, especially when given as amino acid salts, induced propionate production by fecal microbiota, and this effect increased over time. These compounds also reduced the production of acetate such that a consistent increase of the propionate/acetate ratio over time was observed. These effects can readily be explained by a compound-driven expansion of propionate-producing taxa, which we disclose here as well. Moreover, combinations of omega-3 salts with L-rhamnose resulted in even stronger effects on propionate levels and the microbiota, which exceeded the effects of any of the compounds when given individually. Therefore, according to the present invention, the two compounds are applied together.

L-rhamnose is either in its monomeric form or contained in a naturally occurring or synthetic polymer such as rhamnogalacturonan, pectins, hemicellulose, arabic gum, karaya gum, bacterial polysaccharides, ulvan, rhamnolipids, and glycosides naringin, hesperidin, quercitrin.

It is preferable, when the polymer is pectin, preferably from apple or citrus fruits. Pears, apples, guavas, quince, plums, gooseberries, and oranges and other citrus fruits contain large amounts of pectin, while soft fruits, like cherries, grapes, and strawberries, contain small amounts of pectin. Typical amounts of pectin in fresh fruits are 1-1.5% in apples and around 30% in citrus peels. Therefore, the use of pectin from dried citrus peels or apple pomace is preferred, which both are by-products of juice production.

In a preferred configuration, the preparation comprises L-rhamnose in its monomeric form and the omega-3 fatty acids EPA and DHA in the form of fatty acid salts having an organic counter ion selected from lysine, arginine, ornithine, choline and mixtures of the same, or mixtures of free fatty acids and omega-3 fatty acid salts having an organic counter ion selected from lysine, arginine, ornithine, choline. It is preferred when the organic counter ions are selected from lysine, arginine and ornithine. It is particularly preferred to use lysine-salts of EPA and DHA.

In another preferred configuration, the preparation comprises L-rhamnose in a polymeric form and the omega-3 fatty acids EPA and DHA in the form of fatty acid salts having an organic counter ion selected from lysine, arginine, ornithine, choline and mixtures of the same. The polymeric form of L-rhamnose is preferably pectin, more preferably from apple or citrus fruits.

In an alternative embodiment, the preparation according to the present invention further comprises one or more of the following: probiotic strains, preferably selected from *Propionibacterium, Veillonella alcalescens, Clostridium propionicum, Selenomonas, Micromonospora, Bacteroides,* and *Ruminococcus, Bacillus,* preferably selected from *B. subtilis, B. licheniformis,* or B. *amyloliquefaciens,* prebiotics, amino acids, amino acid salts, peptides.

The preparation may further comprise comprising substances that stimulate the production of propionate, preferably pectin or rhamnogalacturonan, laminarin, galactomannan, barley β-glucan, pyrodextrin, pullulan, arabinoxylan, inulin, fructo-oligosaccharides, galacto-oligosaccharides.

In an advantageous configuration, the preparation according to the present invention comprises at least 10 weight-% of omega-3 fatty acid, preferably at least 20 weight-%, more preferably at least 30 weight-%, most preferably at least 40 weight-% omega-3 fatty acid.

In another advantageous configuration, the preparation comprises at least 10 weight-% of L-rhamnose, preferably at least 20 weight-%, more preferably at least 30 weight-%, most preferably at least 40 weight-% of L-rhamnose.

Another aspect of the present invention refers to a pharmaceutical or nutraceutical dosage form comprising a preparation according to the present invention, wherein the dosage form is a tablet or capsule and the total weight of L-rhamnose, EPA and DHA is not more than 5 g, preferably not more than 3 g, more preferably not more than 1 g.

In an alternative configuration, the pharmaceutical or nutraceutical dosage form comprises granules, sprinkles or sachets and the total weight of L-rhamnose, EPA and DHA is not more than 50 g, preferably not more than 30 g, more preferably not more than 10 g.

Another important aspect of the invention is the application of the compounds in a targeted-release formulation. This allows L-rhamnose and omega-3 fatty acids to act in synergism at high concentrations in the large intestine, because omega-3 fatty acids would otherwise be absorbed in the small intestine if applied without a specific colon-targeted release formulation.

Therefore, in a preferred configuration of the present invention, the compounds are combined in a formulation for enteral delivery that provides protection against gastric conditions and that provides targeted release of the preparation in the large intestine. The targeted-release formulation can be obtained by adding enteric polymers to the matrix of the dosage form, or by adding a coating to the dosage form, preferably an enteric coating. Alternatively, a colon-specific delivery system may be applied, which enables direct delivery into the colon.

An enteric coating is a barrier applied on oral medication that prevents its dissolution or disintegration in the gastric environment. Most enteric coatings work by presenting a surface that is stable at the intensely acidic pH found in the stomach but breaks down rapidly at a higher pH (alkaline pH). For example, they will not dissolve in the gastric acids of the stomach (pH ~3), but they will start to dissolve in the environment present in the distal small intestine (pH range proximal to distal small intestine is -5.6 to 7.4) [17].

The present invention therefore also refers to a pharmaceutical or nutraceutical dosage form comprising a preparation according to the present invention, further comprising a colon-specific delivery system, preferably a coating, preferably selected from methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein.

As an enteric coating it is preferred to use a polymer polymerized from 10 to 30 % by weight methyl methacrylate, 50 to 70 % by weight methyl acrylate and 5 to 15 % by weight methacrylic acid.

The polymer dispersion as disclosed may preferably comprise 15 to 50 % by weight of a polymer polymerized from 20 to 30 % by weight methyl methacrylate, 60 to 70 % by weight methyl acrylate and 8 to 12 % by weight methacrylic acid. Most preferred the polymer is polymerized from 25 % by weight methyl methacrylate, 65 % by weight methyl acrylate and 10 % by weight methacrylic acid.

A 30 % by weight aqueous dispersion of a polymer polymerized from 25 % by weight methyl methacrylate, 65 % by weight methyl acrylate and 10 % by weight methacrylic acid corresponds to the commercial product EUDRAGUARD^{®} biotic.

The percentages of the monomers add up to 100 %. The functional polymer is applied in amounts of 2-30 mg/cm², preferably 5-20 mg/cm².

In a further preferred configuration of the pharmaceutical or nutraceutical dosage form, the coating comprises pectin or a pectin salt, preferably calcium pectinate. The coating may further comprise pH-dependent polymers or biodegradable polymers, preferably selected from methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein.

One subject of the present invention is a preparation according to the present invention for use as a feed or food supplement or in foodstuffs. Preferred foodstuffs according to the invention are chocolate products, gummies, mueslis, muesli bars, breads and other cereal products, powdered or ready-to-drink formula diets, weight management formulas, milk, yogurts or other dairy products.

A further subject of the present invention is a feed- or foodstuff composition containing a preparation according to the present invention and at least one further feed or food ingredient, preferably selected from proteins, carbohydrates, fats, further probiotics, prebiotics, enzymes, vitamins, immune modulators, milk replacers, minerals, amino acids, coccidiostats, acid-based products, medicines, and combinations thereof.

Where a preparation according to the present invention is combined with probiotics, these are preferably selected from propionate-producing taxa such as *Propionibacterium, Veillonella alcalescens, Clostridium propionicum, Selenomonas, Micromonospora, Bacteroides,* and *Ruminococcus.*

Alternatively, probiotics are selected from the genus *Bacillus;* the Bacilli are preferably selected from the species B. *subtilis, B. licheniformis,* or B. *amyloliquefaciens;* and preferred strains thereof are B. *subtilis* DSM 32315, *B. licheniformis* DSM 32314, or B. *amyloliquefaciens* CECT5940. The cells of the strains of the current invention may be present, in particular in the compositions of the current invention, as spores (which are dormant), as vegetative cells (which are growing), as transition state cells (which are transitioning from growth phase to sporulation phase) or as a combination of at least two, in particular all of these types of cells.

The feed- or foodstuff composition according to the present invention does also include dietary supplements in the form of a pill, capsule, tablet, granule or liquid.

A further subject of the current invention is a pharmaceutical composition containing a preparation according to the present invention and a pharmaceutically acceptable carrier.

The present invention is also directed to a preparation as described above for use as a medicament, preferably for: cardiovascular health, metabolic health, glucose and lipid metabolism, insulin sensitivity, liver health, weight management / weight loss / control, neuronal health.

Preferred is preparation according to the present invention for use in the treatment or prevention of a disease or disorder selected from hyperlipidemia, hypercholesterolemia, non-alcoholic fatty liver, hepatitis, type 2 diabetes, prediabetes, glucose intolerance, arteriosclerosis, other vascular diseases, obesity, adipositas, multiple sclerosis.

### Working examples

### Intestinal screening model

To determine the effect of a EPA/DHA lysine salt (EPA/DHA-Lys) on adult colonic microbiota, an intestinal screening model was used (I-screen, TNO, the Netherlands). Therefore, the I-screen model was inoculated with standard human adult fecal microbiota material, which consisted of pooled fecal donations from six healthy adult volunteers (Caucasian, European lifestyle and nutrition). The fecal material was mixed and grown in a fed-batch fermenter for 40 hours to create a standardized microbiota as described previously [18]. These standard adult gut microbiota sets were stored at - 80°C in 12% glycerol.

The intestinal microbiota was cultured *in vitro* in modified standard ileal efflux medium (SIEM), the composition of which was described by Minekus et al. [19]. All components were supplied by Trititium Microbiology (Veldhoven, The Netherlands). The pH of the medium was adjusted to 5.8.

For the I-screen fermentations, the pre-cultured standardized fecal inoculum was diluted 50 times in modified SIEM. EPA/DHA-Lys was introduced into the I-screen to final concentrations of 1.4 mg/ml and 1.5 mg/ml, respectively; omega-3 ethyl ester and fish oil at 1.4 mg/ml each. Inulin was added as a control at a final concentration of 4 mg/ml. The I-screen incubation was performed under following gas conditions: 0.2% O₂, 0.2% CO₂, 10% H₂, 89.6% N₂. All experiments were carried out in triplicates.

### SCFA analysis

For the analysis of short-chain fatty acids in exposed material from the I-screen, samples were centrifuged (~4000 g, 5 min), clear supernatant was filter sterilized (0.45 µm) and a mixture of formic acid (20%), methanol and 2-ethyl butyric acid (internal standard, 2 mg/ml in methanol) was added. A 3-µL sample with a split ratio of 75.0 was injected on a GC-column (ZB-5HT inferno, ID 0.52 mm, film thickness 0.10 µm; Zebron; Phenomenex, USA) in a Shimadzu GC-2014 gas chromatograph. SCFA parameters analyzed were: acetic acid and propionic acid.

### Polyunsaturated fatty acid compositions

In the examples for the present invention, different polyunsaturated fatty acid compositions were used. Different omega-3 fatty acid salts having an organic counter ion selected from the basic amino acids lysine, arginine and ornithine were prepared. The omega-3 fatty acids Eicosapentaenoic acid (C20:5w3c) (EPA) and Docosahexaenoic acid (C22:6w3c) (DHA) are present in a ratio of around 2:1 (ratio EPA : DHA).

The omega-3 lysine salt (omega-3-lys) contains around 32 weight-% of L-lysine and around 65 weight-% of polyunsaturated fatty acids. The major polyunsaturated fatty acids in the composition are the omega-3 fatty acids Eicosapentaenoic acid (C20:5w3c) (EPA) and Docosahexaenoic acid (C22:6w3c) (DHA), summing up to around 58 weight-% of the composition. The composition also contains minor amounts of Docosaenoic acid isomer (incl. erucic acid) (C22:1), Docosapentaenoic acid (C22:5w3c) and of the omega-6 fatty acids Arachidonic acid (C20:4w6) and Docosatetraenoic acid (C22:4w6c).

The omega-3 arginine salt (omega-3-arg) contains around 35 weight-% of L-arginine and around 64 weight-% of polyunsaturated fatty acids. The major polyunsaturated fatty acids in the composition are the omega-3 fatty acids Eicosapentaenoic acid (C20:5w3c) (EPA) and Docosahexaenoic acid (C22:6w3c) (DHA), summing up to around 49 weight-% of the composition. The composition also contains minor amounts of Docosaenoic acid isomer (incl. erucic acid) (C22:1), Docosapentaenoic acid (C22:5w3c) and of the omega-6 fatty acids Arachidonic acid (C20:4w6) and Docosatetraenoic acid (C22:4w6c).

The omega-3 ornithine salt (omega-3-orn) contains around 29 weight-% of L-ornithine and around 70 weight-% of polyunsaturated fatty acids. The major polyunsaturated fatty acids in the composition are the omega-3 fatty acids Eicosapentaenoic acid (C20:5w3c) (EPA) and Docosahexaenoic acid (C22:6w3c) (DHA), summing up to around 54 weight-% of the composition. The composition also contains minor amounts of Docosaenoic acid isomer (incl. erucic acid) (C22:1), Docosapentaenoic acid (C22:5w3c) and of the omega-6 fatty acids Arachidonic acid (C20:4w6) and Docosatetraenoic acid (C22:4w6c).

### Example 1: EPA/DHA-Lys modulates the production of acetate and propionate by human fecal microbiota

The effect of different omega-3 fatty acid forms on production of the SCFAs acetate, propionate and n-butyrate by human fecal microbiota was analyzed, which is shown in table 1.

**Table 1: Omega-3-lys has unique effects on SCFA production by microbiota. Effects of Omega-3-lys, Omega-3 fish oil, Omega-3 ethyl ester, and inulin on concentrations of acetate, propionate, and n-butyrate after 24 h incubation in human fecal microbiota are given as change in mM compared to a control sample (controls had ~ 40 mM acetate and ~ 8 mM propionate after the 24 h cultivation period). Values are given as means of triplicate experiments.**

| Compound | Acetate | Propionate | n-Butyrate |
|---|---|---|---|
| Omega-3-lys | -3,21 | +1,38 | -0,86 |
| Omega-3 ethyl ester | -2,92 | -0,02 | -1,26 |
| Fish oil | -2,30 | -0,17 | -0,60 |
| Inulin | +5,87 | +0,03 | +0,46 |

The lysine salt of omega-3 (omega-3-lys), but not the commonly used esterified omega-3 derivatives omega-3 ethyl ester (EE) or fish oil, as well as the prebiotic control substance inulin, increased the production of propionate by intestinal microbiota. In parallel, omega-3-lys caused a stronger decrease of acetate production than omega-3 EE or fish oil.

### Example 2: Omega-3-lys, -arg, and -orn salts increase propionate formation and propionate-to-acetate ratio in a human intestinal microbiota

The effect of different omega-3 fatty acid salts on production of the SCFAs acetate, propionate and n-butyrate by human intestinal microbiota was analyzed, which is shown in table 2.

**Table 2: Effects of omega-3 amino acid salts and controls on even-chain SCFA levels in a human intestinal microbiota. Compounds were applied at the following concentrations: Omega-3 amino acid salts = 1,4 mg/ml; EPA/DHA FFA = 0,96 mg/ml; L-lysine = 0,49 mg/ml; L-arginine = 0,52 mg/ml; L-ornithine = 0,5 mg/ml. Values are given as change in mM compared to a control sample as mean of triplicate experiments.**

| Compound | Acetate | Propionate | n-Butyrate | Propionate/acetate ratio |
|---|---|---|---|---|
| Omega-3-lys | -1,26 | +2,28 | +0,06 | 0,69 |
| Omega-3-arg | -0,32 | +2,22 | +0,43 | 0,64 |
| Omega-3-orn | +4,76 | +1,55 | +0,27 | 0,45 |
| FFA | -6,19 | +0,15 | -0,69 | 0,83 |
| Lys | +8,81 | +2,12 | +0,29 | 0,40 |
| Arg | +8,29 | +2,02 | +0,71 | 0,40 |
| Orn | +10,15 | +1,74 | +0,19 | 0,36 |

The effects of different omega-3 amino acid salts omega-3-lysine (omega-3-lys), omega-3-arginine (omega-3-arg) and omega-3-ornithine (omega-3-orn) and their respective controls (free fatty acids (FFA), amino acids lysine (lys), arginine (arg) and ornithine (orn)) on SCFA production by an intestinal microbiota were analyzed. All tested omega-3 salts induced propionate production and increased propionate-to-acetate ratios, with omega-3-lys salt showing strongest effects. The amino acids alone also induced propionate, but at the same time strongly induced acetate, resulting in lower propionate-to-acetate ratios as compared to the omega-3 amino acid salts. Omega-3 FFA alone caused a slight increase of propionate levels and a severe reduction of acetate levels, which resulted in a high propionate-to-acetate ratio. In conclusion, a concomitant increase of propionate production and propionate-to-acetate ratio was only achieved after supplementation with omega-3 amino acid salts, with the lysine salt showing the strongest effect.

### Example 3: A combination of L-rhamnose and EPA/DHA-Lys results in enhanced production of propionate, increase in the propionate-to-acetate ratio, which further enhances over time, by human fecal microbiota 1

To confirm the results for omega-3 amino acid salts, using omega-3 lysine as an example, an additional, independent and more advanced model of fermentation by the human colonic microbiota was applied, the TIM-2 model. Next to omega-3 lysine the prebiotic carbohydrate L-rhamnose was also applied.

### TIM-2 model

TIM-2 is an abbreviation of TNO's in vitro model-2, an advanced dynamic, computer-controlled in vitro model of the adult human colon. TIM-2 simulates to a high degree the successive dynamic processes in the colon and is predictive for what happens in human individuals [19]. The following *in vivo* conditions are simulated in this model: body temperature, pH in the lumen, composition and rate of secretion, delivery of a pre-digested substrate from the 'ileum' (SIEM), mixing and transport of the intestinal contents, absorption of water and microbial metabolites and presence of a complex, high density, metabolically active, anaerobic fecal microbiota of human origin.

The model was set-up and inoculated with the microbiota on the first day. Following an overnight adaptation period in SIEM, the test-products were fed at final concentrations of 1,4 mg/ml EPA/DHA-Lys or 1 mg/ml L-rhamnose in SIEM during an intervention period of 72 hrs. Cultivation in SIEM without any additional substance served as control condition.

Samples were taken from the lumen and the dialysate every 24 hrs (t0, t24, t48 and t72) to study the metabolic activity of the gut microbiota (SCFA production) and changes in composition of the gut microbiota (16S rRNA gene analysis).

### SCFA analysis

Samples were analyzed for SCFA by ion-chromatography.

### Determination of changes in the gut microbiota

Samples for microbiota composition were analyzed by Baseclear (Leiden, the Netherlands), with subsequent bioinformatics analysis by UM using our standard pipeline using the QIIME-package. Briefly, the isolation of genomic DNA from the fecal samples (3mL lumen) was performed using standard molecular biology kits from ZYMO Research provided by BaseClear (Leiden, The Netherlands). The PCR amplification of the 16S rRNA gene (V3 and V4 regions), the Barcoding and the library preparation were carried out by BaseClear. The sequencing was carried out using the Illumina MiSeq system and later the sequences were converted into FASTQ files using BCL2FASTQ pipeline version 1.8.3. The quality cut was applied based on the quality level of Phred (Phred quality score). QIIME software package (1.9.0) was used for microbial analyses. The sequences were classified using Greengenes (version 13.8) as a reference 16S rRNA gene database. Correlations between Operational Taxonomic Units (OTUs) and metabolites or test-product was investigated using by Spearman correlation for metabolites and Kruskal-Wallis correlations for test-products, respectively, by programming in R, using RStudio.

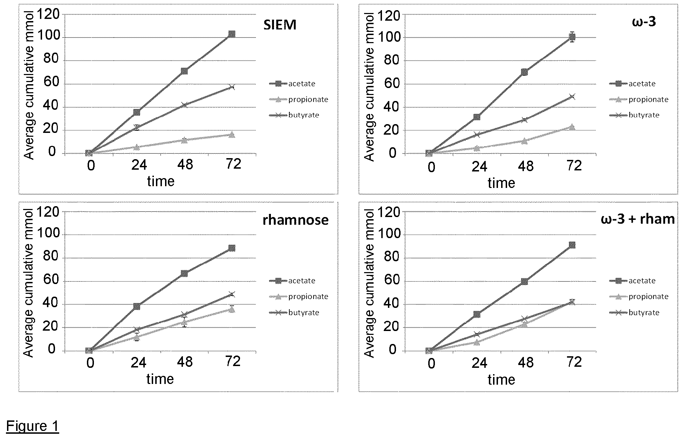

Figure 1 shows the cumulative production of SCFA (given in mmol ± SD) of duplicate experiments, based on samples taken from lumen and dialysate at 0, 24, 48, and 72h. Figure 1 shows accelerated production of propionate by the test compounds, both individually and in combination, whereby the combination of the two leads to highest levels of propionate as well as relatively lower levels of acetate. Table 3 lists absolute values for each data point shown in Figure 1 and states the propionate-to-acetate (Pr/Ac) ratios for each treatment after 24, 48, and 72 hours. The order of Pr/Ac for the 72h treatments is control (SIEM) < L-rhamnose < EPA/DHA-Lys (ω-3) < L-rhamnose + EPA/DHA-Lys (ω-3). Importantly, the combination of L-rhamnose and EPA/DHA-Lys resulted in highest propionate levels as well as Pr/Ac of all test conditions (see tables 3 and 4).

**Table 3: Cumulative production of SCFA in mmol ± SD of duplicate experiments, based on samples taken every 24h from lumen and dialysate.**

| **Control** | | | | | **ω3** | | | | |
|---|---|---|---|---|---|---|---|---|---|
| time | acetate | propionate | butyrate | Pr/Ac | time | acetate | propionate | butyrate | Pr/Ac |
| 0 | 0,00 | **0,00** | 0,00 | | 0 | 0,00 | **0,00** | 0,00 | |
| 24 | 37,90 | **11,15** | 24,37 | **0,29** | 24 | 24,09 | **17,58** | 22,88 | **0,73** |
| 48 | 66,14 | **23,85** | 45,34 | **0,36** | 48 | 42,68 | **35,56** | 41, 23 | **0,83** |
| 72 | 95,52 | **36,90** | 60,99 | **0,39** | 72 | 62,36 | **52,61** | 60, 29 | **0,84** |
| | | | | | | | | | |

| SD | acetate | propionate | butyrate | | time | acetate | propionate | butyrate | |
|---|---|---|---|---|---|---|---|---|---|
| 0 | 0,00 | 0,00 | 0,00 | | 0 | 0,00 | 0,00 | 0,00 | |
| 24 | 0,02 | 0,18 | 2,33 | | 24 | 0,61 | 1 | 0,62 | |
| 48 | 0,34 | 1,25 | 0,86 | | 48 | 2,76 | 0,17 | 0,97 | |
| 72 | 0,42 | 0,16 | 0,37 | | 72 | 4,27 | 0,34 | 0,69 | |
| | | | | | | | | | |

| rhamnose | | | | | ω3 + rhamnose | | | | |
|---|---|---|---|---|---|---|---|---|---|
| time | acetate | propionate | butyrate | Pr/Ac | time | acetate | propionate | butyrate | Pr/Ac |
| 0 | 0,00 | **0,00** | 0,00 | | 0 | 0,00 | **0,00** | 0,00 | |
| 24 | 25,30 | **14,30** | 15,69 | 0,57 | 24 | 23,97 | **15,58** | 16,66 | **0,65** |
| 48 | 56, 28 | **31,64** | 35,02 | 0,56 | 48 | 50,12 | **40,68** | 35,92 | **0,81** |
| 72 | 81,62 | **45,41** | 52,10 | 0,56 | 72 | 76,91 | **72,80** | 55,46 | **0,95** |
| | | | | | | | | | |

| SD | acetate | propionate | butyrate | | time | acetate | propionate | butyrate | |
|---|---|---|---|---|---|---|---|---|---|
| 0 | 0,00 | 0,00 | 0,00 | | 0 | 0,00 | 0,00 | 0,00 | |
| 24 | 1,53 | 3,26 | 0,61 | | 24 | 0,73 | 0,28 | 0,93 | |
| 48 | 0,28 | 4,34 | 0,30 | | 48 | 2,16 | 1,74 | 0,22 | |
| 72 | 0,62 | 2,78 | 1,30 | | 72 | 1,47 | 2,02 | 1,15 | |

**Table 4: Ratios of SCFA average and SD) were calculated by summing acetate+propionate+butyrate and expressing the individual SCFA as a percentage of the sum.**

| **ratios** | | | | |
|---|---|---|---|---|
| | | acetate | propionate | butyrate |
| **Control** | average | 58,3% | 9,3% | 32,4% |
| | SD | 0,0% | 0,1% | 0,1% |
| **ω-3** | average | 58,2% | 13,4% | 28,4% |
| | SD | 0,7% | 2,1% | 1,7% |
| **rhamnose** | average | 51,1% | 20,8% | 28,1% |
| | SD | 0,6% | 1,5% | 0,9% |
| **ω-3 + rhamnose** | average | 52,0% | 24,1% | 23,9% |
| | SD | 0,5% | 0,5% | 0,0% |

### Example 5: Time-dependent modulation of the human fecal microbiota 1 by L-rhamnose and EPA/DHA-Lys individually and in combination: expansion of propionate-producing taxa

The microbiota composition was determined as described under Example 4.

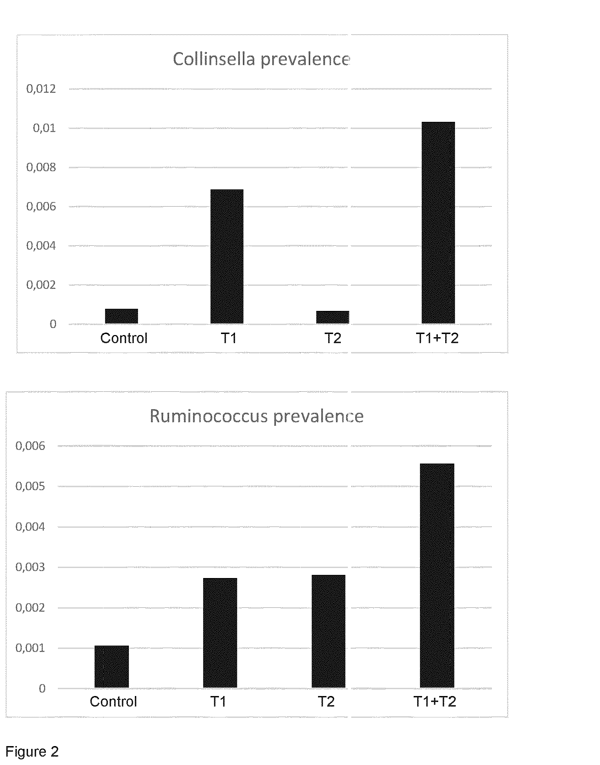

Synergistic effects of the test compounds were found for the taxa *Ruminococcus* and *Collinsella,* as shown in Figure 2.

Figure 2 shows the prevalence of *Collinsella* and *Ruminococcus* after 72h cultivation of human fecal microbiota. T1= EPA/DHA-Lys; T2= L-rhamnose.

### Example 6: A combination of L-rhamnose and EPA/DHA-Lys results in enhanced production of propionate, increase in the propionate-to-acetate ratio, which further enhances over time, by human fecal microbiota 2

A second TIM-2 study was performed, using conditions as described under Example 3 but applying a different human fecal microbiota (termed microbiota 2), to assess whether the observed effects are dependent on the initial microbiota composition.

The results obtained with microbiota 2 demonstrate that it differs from microbiota 1 in terms of composition and activity, reflected by a different production of SCFA in the control group at all time points studied. For example, in microbiota 2 the background propionate production is higher than in microbiota 1. Despite this, all test conditions (EPA/DHA-Lys ± rhamnose) increased the levels of propionate compared to the control group. Compared to microbiota 1, propionate induction and Pr/Ac increase by EPA/DHA-Lys was even stronger and exceeded the effect of L-rhamnose. Moreover, the synergistic effect on both readouts by co-supplementation of the compounds was also confirmed. It can be concluded that omega-3 fatty acids, especially EPA/DHA-Lys and L-rhamnose increase propionate levels and Pr/Ac irrespective of the initial composition and activity of a human fecal microbiota and that they synergistically interact with each other therein (tables 5 and 6).

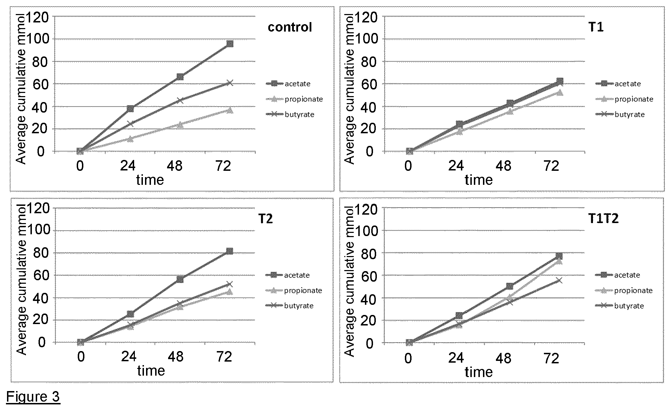

Figure 3 shows the cumulative production of SCFA (given in mmol ± SD) of duplicate experiments, based on samples taken from lumen and dialysate at 0, 24, 48, and 72h. T1= EPA/DHA-Lys (w-3); T2= L-rhamnose.

**Table 5: Ratios of SCFA (average and SD) were calculated by summing acetate+propionate+butyrate and expressing the individual SCFA as a percentage of the sum.**

| | | **Acetate** | **Propionate** | **Butyrate** |
|---|---|---|---|---|
| **Control** | average | 49,4% | 19,1% | 31,5% |
| | SD | 0,3% | 0,1% | 0,4% |
| **w-3 (T1)** | average | 35,7% | 30,1% | 34,3% |
| | SD | 0,6% | 0,3% | 0,9% |
| **rhamnose (T2)** | average | 45,7% | 25,3% | 29,0% |
| | SD | 2,8% | 0,3% | 2,6% |
| **T1 + T2** | average | 37,5% | 35,5% | 27,0% |
| | SD | 3,4% | 0,9% | 4,3% |

**Table 6: Cumulative production of SCFA in mmol ± SD of duplicate experiments, based on samples taken every 24h from lumen and dialysate.**

| **Control** | | | | | **ω3** | | | | |
|---|---|---|---|---|---|---|---|---|---|
| time | acetate | propionate | butyrate | Pr/Ac | time | acetate | propionate | butyrate | Pr/Ac |
| 0 | 0,00 | 0,00 | 0,00 | | | 0,00 | 0,00 | 0,00 | |
| 24 | 37,90 | 11,15 | 24,37 | 0,29 | | 24,09 | 17,58 | 22,88 | 0,73 |
| 48 | 66,14 | 23,85 | 45,34 | 0,36 | | 42,68 | 35,56 | 41,23 | 0,83 |
| 72 | 95,52 | 36,90 | 60,99 | 0,39 | | 62,36 | 52,61 | 60,29 | 0,84 |
| | | | | | | | | | |

| SD | acetate | propionate | butyrate | | time | acetate | propionate | butyrate | |
|---|---|---|---|---|---|---|---|---|---|
| 0 | 0,00 | 0,00 | 0,00 | | 0 | 0,00 | 0,00 | 0,00 | |
| 24 | 0,02 | 0,18 | 2,33 | | 24 | 0,61 | 1,00 | 0,62 | |
| 48 | 0,34 | 1,25 | 0,86 | | 48 | 2,76 | 0,17 | 0,97 | |
| 72 | 0,42 | 0,16 | 0,37 | | 72 | 4,27 | 0,34 | 0,69 | |
| | | | | | | | | | |

| **rhamnose** | | | | **ω3 + rhamnose** | | | | | |
|---|---|---|---|---|---|---|---|---|---|
| time | acetate | propionate | butyrate | Pr/Ac | time | acetate | propionate | butyrate | Pr/Ac |
| 0 | 0,00 | 0,00 | 0,00 | | | 0,00 | 0,00 | 0,00 | |
| 24 | 25,30 | 14,30 | 15,69 | 0,57 | | 23,97 | 15,58 | 16,66 | 0,65 |
| 48 | 56,28 | 31,64 | 35,02 | 0,56 | | 50,12 | 40,68 | 35,92 | 0,81 |
| 72 | 81,62 | 45,41 | 52,10 | 0,56 | | 76,91 | 72,80 | 55,45 | 0,95 |
| | | | | | | | | | |

| SD | acetate | propionate | butyrate | | time | acetate | propionate | butyrate | |
|---|---|---|---|---|---|---|---|---|---|
| 0 | 0,00 | 0,00 | 0,00 | | 0 | 0,00 | 0,00 | 0,00 | |
| 24 | 1,53 | 3,26 | 0,61 | | 24 | 0,73 | 0,28 | 0,93 | |
| 48 | 0,28 | 4,34 | 0,30 | | 48 | 2,16 | 1,74 | 0,22 | |
| 72 | 0,62 | 2,78 | 1,30 | | 72 | 1,47 | 2,02 | 1,15 | |

### Example 7: Time-dependent modulation of the human fecal microbiota 2 by L-rhamnose and EPA/DHA-Lys individually and in combination: expansion of propionate-producing taxa

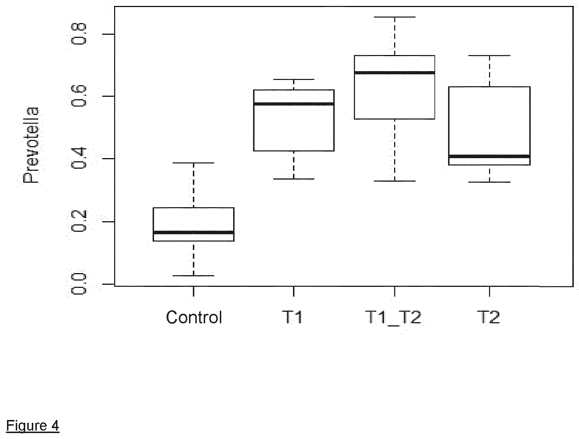

The composition of the gut microbiota was determined as described under Example 3. By using Kruskal-Wallis correlation analysis the genus *Prevotella* was identified to be significantly affected by omega-3 fatty acids, especially EPA/DHA-Lys and by L-rhamnose, both individually and even stronger in combination (Figure 4). The abundances of different Operational taxonomic unit (OTUs) was correlated with levels of microbial short-chain fatty acids. As can be seen in table 7, a strongly positive correlation between *Prevotella* and propionate was identified; positive though weaker correlations occurred also for *Prevotellaceae, Lactobacillus,* and *Clostridium.* It can be concluded that these taxa mediate the omega-3-fatty acid- and L-rhamnose-dependent induction of propionate production by gut microbiota.

Figure 4 shows the prevalence of *Prevotella* under the different experimental conditions; *Prevotella* is positively correlated with omegal-3 fatty acids (q-value = 0.00054) and L-rhamnose (q-value = 0.0060). T1= EPA/DHA-Lys; T2= L-rhamnose. Time points 24, 48, and 72h were included in the analysis.

**Table 7: Rho-correlations between microbial metabolites and abundances of OTUs. Time points 24, 48, and 72h were included in the analysis.**

| **SCFA** | | | |
|---|---|---|---|
| acetate | propionate | butyrate | **OTU** |
| | -0,32 | | Actinomyces |
| | | | Bifidobacterium |
| -0,43 | -0,37 | | Coriobacteriaceae |
| -0,44 | -0,43 | | Adlercreutzia |
| -0,46 | -0,5 | -0,44 | Bacteroidales;Other |
| -0,38 | -0,49 | -0,42 | Bacteroides |
| -0,52 | -0,6 | | Parabacteroides |
| | 0,35 | | Prevotellaceae |
| | 0,58 | | Prevotella |
| -0,58 | -0,72 | -0,41 | Rikenellaceae |
| -0,38 | | -0,41 | Bacteroidales;f |
| -0,44 | -0,52 | | Barnesiellaceae |
| | -0,44 | | Butyricimonas |
| | -0,33 | | Odoribacter |
| -0,48 | -0,51 | -0,58 | Paraprevotella |
| 0,54 | 0,55 | 0,61 | Lactobacillus |
| | | 0,41 | Lactococcus |
| | -0,46 | | Streptococcus |
| | -0,38 | | Turicibacter |
| | -0,42 | | Clostridiales;Other |
| | -0,4 | | Clostridiales;g |
| -0,47 | -0,55 | | Christensenellaceae |
| | -0,33 | | Clostridiaceae;Other |
| 0,39 | 0,47 | | Clostridium |
| | -0,41 | | Clostridiaceae;g |
| | -0,36 | | Lachnospiraceae;Other |
| | | 0,51 | Lachnospiraceae;g_ |
| | -0,5 | | Blautia |
| | -0,43 | | Coprococcus |
| | -0,53 | | Dorea |
| -0,41 | -0,41 | | Lachnobacterium |
| | -0,47 | | Lachnospira |
| | -0,41 | | Roseburia |
| | -0,51 | | Ruminococcus |
| | | | Peptostreptococcaceae |
| | -0,5 | | Ru min ococcace ae; Other |
| -0,46 | -0,54 | | Ruminococcaceae;g_ |
| | -0,32 | | Faecalibacterium |
| | -0,49 | | Oscillospira |
| | -0,55 | | Ruminococcus |
| | -0,32 | | Dialister |
| | -0,43 | | Mogibacteriaceae |
| | -0,58 | | Erysipelotrichaceae |
| | -0,4 | | Holdemania |
| -0,44 | -0,6 | | Eubacterium |
| | -0,44 | | Bilophila |
| | | | Morganella |
| | -0,35 | | Haemophilus |
| | | 0,46 | Acinetobacter |
| | -0,44 | | Anaeroplasmataceae |
| | -0,56 | | Mollicutes |
| | -0,46 | | Akkermansia |

### Example 8: Combinations of polymer-bound L-rhamnose (pectin) and EPA/DHA-Lys results in enhanced production of propionate by a human fecal microbiota

In the following, we assessed whether our observations made with monomeric L-rhamnose can be extended to L-rhamnose contained in naturally occurring polymers with prebiotic functions. One such polymer is pectin, which is found as part of the cell walls of dicotyledonous plants and which contains L-rhamnose in the form of rhamnogalacturonan. Venema et al. reported that fruits have similar L-rhamnose contents ranging from 1,5 - 3 % [20]. Two types of pectin were applied, one sourced from citrus and one from apple, and tested their metabolization by a human colonic microbiota to SCFAs, individually and each in combination with omega-3-lys, over a time course of 72 hours.

### Human intestinal microbiota fermentation model

Effects of pectin were analyzed using a sophisticated system with standardized human fecal microbiota in a pH buffered and temperature controlled high-throughput analytical system with subsequent SCFA analysis.

Human fecal microbiota inoculum was received by five days fed-batch cultivation of a microbiota sample from a single healthy adult donor, with cryoprotected aliquots stored at -80°C. Aliquots were revitalized for 46 hrs under standardized conditions and the resulting inoculum supplied with 7.5 mg/ml pectin (origin: either from apple or citrus) and/or 1,4 mg/ml EPA/DHA-Lys (ω-3) (final concentrations in modified SIEM). Samples were taken from supernatants every 24 hrs (t0, t24, t48 and t72) to study the metabolic activity of the gut microbiota (SCFA production). Cultivation in modified SIEM without any additional substance served as a control.

### SCFA analysis

Samples were analyzed for SCFA by gas-chromatography.

**Table 8: Cumulative production of SCFA in mmol ± SD of triplicate experiments, based on samples taken every 24h from supernatant.**

| **Control** | | | | | **ω3** | | | | |
|---|---|---|---|---|---|---|---|---|---|
| time | acetate | propionate | butyrate | Pr/Ac | time | acetate | propionate | butyrate | Pr/Ac |
| 0 | 0,00 | 0,00 | 0,00 | | 0 | 0,00 | 0,00 | 0,00 | |
| 24 | 18,23 | 5,47 | 2,35 | 0,30 | 24 | 20,98 | 6,84 | 3,22 | 0,33 |
| 48 | 28,98 | 9,45 | 5,67 | 0,33 | 48 | 26,64 | 9,72 | 5,07 | 0,36 |
| 72 | 35,64 | 8,05 | 5,52 | 0,23 | 72 | 36,25 | 12,15 | 7,07 | 0,34 |
| | | | | | | | | | |

| SD | acetate | propionate | butyrate | | time | acetate | propionate | butyrate | |
|---|---|---|---|---|---|---|---|---|---|
| 0 | 0,00 | 0,00 | 0,00 | | 0 | 0,00 | 0,00 | 0,00 | |
| 24 | 0,25 | 0,07 | 0,11 | | 24 | 2,94 | 1,02 | 0,54 | |
| 48 | 3,16 | 0,81 | 0,11 | | 48 | 2,83 | 0,67 | 0,11 | |
| 72 | 0,17 | 5,12 | 0,17 | | 72 | 0,67 | 0,11 | 0,05 | |
| | | | | | | | | | |

| **pectin (apple)** | | | | | **ω3 + pectin (apple)** | | | | |
|---|---|---|---|---|---|---|---|---|---|
| time | acetate | propionate | butyrate | Pr/Ac | time | acetate | propionate | butyrate | Pr/Ac |
| 0 | 0,00 | 0,00 | 0,00 | | 0 | 0,00 | 0,00 | 0,00 | |
| 24 | 44,30 | 8,14 | 4,88 | 0,18 | 24 | 39,80 | 8,68 | 3,44 | 0,22 |
| 48 | 56,01 | 13,99 | 6,51 | 0,25 | 48 | 50,01 | 15,70 | 5,49 | 0,31 |
| 72 | 58,51 | 14,04 | 8,51 | 0,24 | 72 | 53,79 | 16,87 | 7,38 | 0,31 |
| | | | | | | | | | |

| SD | acetate | propionate | butyrate | | time | acetate | propionate | butyrate | |
|---|---|---|---|---|---|---|---|---|---|
| 0 | 0,00 | 0,00 | 0,00 | | 0 | 0,00 | 0,00 | 0,00 | |
| 24 | 0,83 | 0,06 | 0,00 | | 24 | 3,76 | 0,17 | 0,23 | |
| 48 | 2,44 | 0,45 | 0,11 | | 48 | 5,50 | 1,05 | 0,14 | |
| 72 | 2,52 | 1,05 | 1,37 | | 72 | 2,91 | 0,58 | 1,11 | |
| | | | | | | | | | |

| **pectin (citrus)** | | | | | **ω3 + pectin (citrus)** | | | | |
|---|---|---|---|---|---|---|---|---|---|
| time | acetate | propionate | butyrate | Pr/Ac | time | acetate | propionate | butyrate | Pr/Ac |
| 0 | 0,00 | 0,00 | 0,00 | | 0 | 0,00 | 0,00 | 0,00 | |
| 24 | 36,08 | 7,11 | 8,40 | 0,20 | 24 | 35,97 | 8,10 | 7,45 | 0,23 |
| 48 | 43,57 | 11,16 | 8,10 | 0,26 | 48 | 47,18 | 13,95 | 8,02 | 0,30 |
| 72 | 54,40 | 12,51 | 10,37 | 0,23 | 72 | 49,35 | 14,62 | 8,51 | 0,30 |
| | | | | | | | | | |

| SD | acetate | propionate | butyrate | | time | acetate | propionate | butyrate | |
|---|---|---|---|---|---|---|---|---|---|
| 0 | 0,00 | 0,00 | 0,00 | | 0 | 0,00 | 0,00 | 0,00 | |
| 24 | 4,11 | 0,70 | 0,72 | | 24 | 0,49 | 0,72 | 0,65 | |
| 48 | 12,09 | 1,76 | 2,44 | | 48 | 7,49 | 1,07 | 0,70 | |
| 72 | 1,45 | 0,23 | 0,43 | | 72 | 4,14 | 0,34 | 0,81 | |
| | | | | | | | | | |

Table 8 shows the cumulative production of SCFA (given in mmol ± SD) of triplicate experiments, based on samples taken from supernatant at 0, 24, 48, and 72h. Table 8 shows increased production of propionate by omega-3-lys (ω-3) compared to control at each tested time point (24, 48, 72 h), and consistently higher propionate-to-acetate ratios as found in the control samples.

**Table 9: Ratios of SCFA (average and SD) were calculated by summing acetate+propionate+butyrate and expressing the individual SCFA as a percentage of the sum.**

| **time: 72h** | **%** | **Acetate** | **Propionate** | **Butyrate** |
|---|---|---|---|---|
| **Control** | average | 72,41 | 16,37 | 11,22 |
| | SD | 0,17 | 0,03 | 0,21 |
| **ω3** | average | 65,34 | 21,90 | 12,75 |
| | SD | 0,51 | 0,41 | 0,10 |
| **pectin (apple)** | average | 72,18 | 17,32 | 10,50 |
| | SD | 0,55 | 0,34 | 0,20 |
| **ω3 + pectin (apple)** | average | 68,92 | 21,62 | 9,45 |
| | SD | 1,56 | 0,41 | 1,15 |
| **pectin (citrus)** | average | 70,40 | 16,19 | 13,41 |
| | SD | 0,08 | 0,34 | 0,26 |
| **ω3 + pectin (citrus)** | average | 68,08 | 20,18 | 11,74 |
| | SD | 0,77 | 1,03 | 0,26 |

In summary, the pectin-treated samples showed higher propionate levels than control after (24, 48, 72 h). Pr/Ac ratios were lower than or similar to control, though, reflecting higher acetate concentrations found in both pectin-treated samples. Importantly, co-incubations of citrus- as well as apple-pectin with omega-3-lys resulted in propionate levels that surpassed those of the single treatments at each time point. Again, Pr/Ac ratios were in between those of the control and single treatments, reflecting the higher contribution of pectins to acetate production (also displayed in the percentage distribution of SCFA shown in table 9).

Similar results were obtained by using a different SIEM medium with a minimal carbohydrate content.

These results broaden our discovery, accordingly L-rhamnose, either when applied as a monomer or when contained in a naturally occurring carbohydrate polymer, as exemplified for pectin, has a synergistic effect on omega-3- dependent production of propionate by a human colonic microbiota.

### Example 9: Capsules comprising EPA-DHA amino acid salts and L-rhamnose as food supplement

The following components (as shown in table 10) were filled in HPMC capsules (size 0).

**Table 10: Preparations for filling into HPMC capsules. *Amino acids are selected from L-ornithine, L-lysine and L-arginine.**

| Compound | Capsule I | Capsule II | Capsule III |
|---|---|---|---|
| Omega-3 amino acid* salt | 250 mg | 50 mg | 800 mg |
| L-rhamnose | 200 mg | 50 mg | 800 mg |
| L-ornithine L-aspartate | 200 mg | 50 mg | 800 mg |
| Pectin | 250 mg | 50 mg | 800 mg |
| Choline | 82,5 mg | 82,5 mg | 82,5 mg |

The capsules may further contain amino acids selected from L-ornithine, L-aspartate, L-lysine and L-arginine.

The capsules may further contain further carbohydrate ingredients, selected from arabinoxylans, barley grain fibre, oat grain fibre, rye fibre, wheat bran fibre, inulins, fructooligosaccharides (FOS), galactooligosaccharides (GOS), resistant starch, beta-glucans, glucomannans, galactoglucomannans, guar gum and xylooligosaccharides.

The capsules may further contain one or more plant extracts, selected from ginger, cinnamon, grapefruit, parsley, turmeric, curcuma, olive fruit, panax ginseng, horseradish, garlic, broccoli, spirulina, pomegranate, cauliflower, kale, cilantro, green tea, onions, and milk thistle.

The capsules may further contain charcoal, chitosan, glutathione, monacolin K, plant sterols, plant stanols, sulforaphane, collagen, hyalurone.

The capsules may comprise further vitamins selected from biotin, vitamin A, vitamin B1 (thiamine), vitamin B2 (riboflavin), vitamin B3 (niacin), vitamin B5 (pantothenic acid), vitamin B9 (folic acid or folate), vitamin C (ascorbic acid), vitamin D (calciferols), vitamin E (tocopherols and tocotrienols) and vitamin K (quinones) or minerals selected from sulfur, iron, chlorine, calcium, chromium, cobalt, copper, magnesium, manganese, molybdenum, iodine, selenium, and zinc.

### Example 10: Capsules comprising EPA-DHA amino acid salts, L-rhamnose, enteric coating

The capsules as prepared in example 9 were coated with an enteric coating composition as colon-specific delivery system (as shown in table 11).

**Table 11: Coating composition**

| Compound | Dry substance [g] | Content based on coating [%] | Weight gain [%] | Content based on capsule [%] |
|---|---|---|---|---|
| EUDRAGUARD^{®} biotic | 40,8 | 36,9 | 8,2 | 6,7 |
| HPMC | 43,1 | 39,0 | 8,6 | 7,1 |
| Talc | 20,4 | 18,4 | 4,0 | 3,3 |
| Polyethylene glycol | 4,3 | 3,9 | 0,9 | 0,7 |
| Triethyl citrate | 2,0 | 1,8 | 0,4 | 0,3 |

### Example 11: Formulations comprising EPA-DHA amino acid salts with pectin-based delivery systems (reference example)

The following components (as listed in table 12) were mixed and either filled in HPMC capsules (size 0) or subjected to microencapsulation.

**Table 12: Preoarations for fillina into HPMG capsules or microencaosulation. Amino acids are selected from L-ornithine, L-lysine and L-arginine.**

| Compound | Capsule / Mixture I | Capsule / Mixture II | Capsule / Mixture III |
|---|---|---|---|
| Omega-3 amino acid* salt | 250 mg | 50 mg | 800 mg |
| L-ornithine L-aspartate | 200 mg | 50 mg | 800 mg |
| Choline | 82,5 mg | 82,5 mg | 82,5 mg |

Capsules I - III were coated with compositions containing pectin or calcium pectinate.

Mixtures I - III were microencapsulated with compositions containing pectin or calcium pectinate.

The coating compositions for capsules or mixtures may further contain pH dependent polymers or biodegradable polymers, preferably selected from chitosan, gelatin, HPMC methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein.

The capsules and mixtures may further contain L-rhamnose and ingredients listed in Example 9.

### References

1. Gibson GR, Hutkins R, Sanders ME, Prescott SL, Reimer RA, Salminen SJ, Scott K, Stanton C, Swanson KS, Cani PD et al: Expert consensus document: The International Scientific Association for Probiotics and Prebiotics (ISAPP) consensus statement on the definition and scope of prebiotics. Nat Rev Gastroenterol Hepatol 2017, 14(8):491-502.
2. Hill C, Guarner F, Reid G, Gibson GR, Merenstein DJ, Pot B, Morelli L, Canani RB, Flint HJ, Salminen S et al: Expert consensus document. The International Scientific Association for Probiotics and Prebiotics consensus statement on the scope and appropriate use of the term probiotic. Nat Rev Gastroenterol Hepatol 2014, 11(8):506-514.
3. Riviere A, Selak M, Lantin D, Leroy F, De Vuyst L: Bifidobacteria and Butyrate-Producing Colon Bacteria: Importance and Strategies for Their Stimulation in the Human Gut. Front Microbiol 2016, 7:979.
4. Boets E, Gomand SV, Deroover L, Preston T, Vermeulen K, De Preter V, Hamer HM, Van den Mooter G, De Vuyst L, Courtin CM et al: Systemic availability and metabolism of colonic-derived short-chain fatty acids in healthy subjects: a stable isotope study. J Physiol 2017, 595(2):541-555**.**
5. Wolever TM, Fernandes J, Rao AV: Serum acetate:propionate ratio is related to serum cholesterol in men but not women. J Nutr 1996, 126(11):2790-2797.
6. Chen WJ, Anderson JW, Jennings D: Propionate may mediate the hypocholesterolemic effects of certain soluble plant fibers in cholesterol-fed rats. Proc Soc Exp Biol Med 1984, 175(2):215-218.
7. Demigne C, Morand C, Levrat MA, Besson C, Moundras C, Remesy C: Effect of propionate on fatty acid and cholesterol synthesis and on acetate metabolism in isolated rat hepatocytes. Br J Nutr 1995, 74(2):209-219.
8. Nishina PM, Freedland RA: Effects of propionate on lipid biosynthesis in isolated rat hepatocytes. J Nutr 1990, 120(7):668-673**.**
9. Weitkunat K, Schumann S, Nickel D, Kappo KA, Petzke KJ, Kipp AP, Blaut M, Klaus S: Importance of propionate for the repression of hepatic lipogenesis and improvement of insulin sensitivity in high-fat diet-induced obesity. Mol Nutr Food Res 2016, 60(12):2611-2621.
10. Weitkunat K, Schumann S, Nickel D, Hornemann S, Petzke KJ, Schulze MB, Pfeiffer AF, Klaus S: Odd-chain fatty acids as a biomarker for dietary fiber intake: a novel pathway for endogenous production from propionate. Am J Clin Nutr 2017, 105(6):1544-1551.
11. Chambers ES, Viardot A, Psichas A, Morrison DJ, Murphy KG, Zac-Varghese SE, MacDougall K, Preston T, Tedford C, Finlayson GS et al: Effects of targeted delivery of propionate to the human colon on appetite regulation, body weight maintenance and adiposity in overweight adults. Gut 2015, 64(11):1744-1754.
12. Hoverstad T, Midtvedt T: Short-chain fatty acids in germfree mice and rats. J Nutr 1986, 116(9):1772-1776.
13. Weitkunat K, Schumann S, Petzke KJ, Blaut M, Loh G, Klaus S: Effects of dietary inulin on bacterial growth, short-chain fatty acid production and hepatic lipid metabolism in gnotobiotic mice. J Nutr Biochem 2015, 26(9):929-937.
14. Smith EA, Macfarlane GT: Dissimilatory amino Acid metabolism in human colonic bacteria. Anaerobe 1997, 3(5):327-337.
15. Vogt JA, Pencharz PB, Wolever TM: L-Rhamnose increases serum propionate in humans. Am J Clin Nutr 2004, 80(1):89-94.
16. Darzi J, Frost GS, Swann JR, Costabile A, Robertson MD: L-rhamnose as a source of colonic propionate inhibits insulin secretion but does not influence measures of appetite or food intake. Appetite 2016, 98:142-149.
17. Abuhelwa AY, Foster DJ, Upton RN: A Quantitative Review and Meta-Models of the Variability and Factors Affecting Oral Drug Absorption-Part I: Gastrointestinal pH. AAPS J 2016, 18(5):1309-1321.
18. Ladirat SE, Schols HA, Nauta A, Schoterman MH, Keijser BJ, Montijn RC, Gruppen H, Schuren FH: High-throughput analysis of the impact of antibiotics on the human intestinal microbiota composition. J Microbiol Methods 2013, 92(3):387-397.
19. Minekus M, Smeets-Peeters M, Bernalier A, Marol-Bonnin S, Havenaar R, Marteau P, Alric M, Fonty G, Huis in't Veld JH: A computer-controlled system to simulate conditions of the large intestine with peristaltic mixing, water absorption and absorption of fermentation products. Appl Microbiol Biotechnol 1999, 53(1):108-114.
20. Larsen N, Bussolo de Souza C, Krych L, Barbosa Cahu T, Wiese M, Kot W, Hansen KM, Blennow A, Venema K, Jespersen L: Potential of Pectins to Beneficially Modulate the Gut Microbiota Depends on Their Structural Properties. Front Microbiol 2019, 10:223.

## Claims

1. A preparation comprising
a) L-rhamnose either in its monomeric form or contained in a naturally occurring or synthetic polymer, and
b) the omega-3 fatty acids eicosapentaenoic acid (EPA) and docosahexaenoic acid (DHA) in the form of either
fatty acid salts having an organic counter ion selected from lysine, arginine, ornithine, choline and mixtures of the same, or
mixtures of free fatty acids and omega-3 fatty acid salts having an organic counter ion selected from lysine, arginine, ornithine, choline:
wherein component a) and b) form a mixture in the preparation.

2. Preparation according to claim 1, wherein L-rhamnose is either in its monomeric form or contained in a naturally occurring or synthetic polymer selected from pectins, hemicellulose, arabic gum, karaya gum, bacterial polysaccharides, ulvan, rhamnolipids, and glycosides naringin, hesperidin, or quercitrin.

3. Preparation according to claim 2, wherein the polymer is pectin, preferably from apple or citrus fruits.

4. Preparation according to any one of the preceding claims, wherein EPA and DHA are in the form of fatty acid salts having an organic counter ion selected from lysine, arginine, ornithine, choline and mixtures of the same, preferably selected from lysine, arginine and ornithine.

5. Preparation according to any one of the preceding claims, further comprising one or more of the following: probiotic strains, preferably selected from *Propionibacterium, Veillonella alcalescens, Clostridium propionicum, Selenomonas, Micromonospora, Bacteroides,* and *Ruminococcus, Bacillus,* preferably selected from B. *subtilis, B. licheniformis,* or B. *amyloliquefaciens,* prebiotics, amino acids, amino acid salts, peptides.

6. Preparation according to any one of the preceding claims, further comprising one or more of the following: pectin, rhamnogalacturonan, laminarin, galactomannan, barley β-glucan, pyrodextrin, pullulan, arabinoxylan, inulin, fructo-oligosaccharides, galacto-oligosaccharides.

7. Preparation according to any one of the preceding claims, comprising at least 10 weight-% of omega-3 fatty acid, preferably at least 20 weight-%, more preferably at least 30 weight-%, most preferably at least 40 weight-% omega-3 fatty acid.

8. Preparation according to any one of the preceding claims, comprising at least 10 weight-% of L-rhamnose, preferably at least 20 weight-%, more preferably at least 30 weight-%, most preferably at least 40 weight-% of L-rhamnose.

9. A pharmaceutical or nutraceutical dosage form comprising a preparation according to any one of the preceding claims, wherein the dosage form is a tablet or capsule and the total weight of L-rhamnose, EPA and DHA is not more than 5 g, preferably not more than 3 g, more preferably not more than 1 g.

10. A pharmaceutical or nutraceutical dosage form comprising a preparation according to any one of claims 1 to 8, wherein the dosage form comprises granules, sprinkles or sachets and the total weight of L-rhamnose, EPA and DHA is not more than 50 g, preferably not more than 30 g, more preferably not more than 10 g.

11. A pharmaceutical or nutraceutical dosage form according to claim 9 or 10 further comprising a colon-specific delivery system, preferably comprising a coating, preferably selected from methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein.

12. Pharmaceutical or nutraceutical dosage form according to claim 11, wherein the coating comprises pectin or a pectin salt, preferably calcium pectinate.

13. A feed- or foodstuff composition containing a preparation according to one of claims 1 to 8 and at least one further feed or food ingredient, preferably selected from proteins, carbohydrates, fats, further probiotics, prebiotics, enzymes, vitamins, immune modulators, milk replacers, minerals, amino acids, coccidiostats, acid-based products, medicines, and combinations thereof.

14. A preparation according to one of claims 1 to 8 for use as a feed or food supplement or in a pharmaceutical composition.

15. A preparation according to any one of claims 1 to 8 for use as a medicament, preferably for: cardiovascular health, metabolic health, glucose and lipid metabolism, insulin sensitivity, liver health, weight management / weight loss / control, neuronal health.

16. A preparation according to any one of claims 1 to 8 for use in the treatment or prevention of a disease or disorder selected from hyperlipidemia, hypercholesterolemia, non-alcoholic fatty liver, hepatitis, type 2 diabetes, prediabetes, glucose intolerance, arteriosclerosis, other vascular diseases, obesity, adipositas, multiple sclerosis.

## Patentansprüche

1. Zubereitung, umfassend
a) L-Rhamnose entweder in dessen monomerer Form oder enthalten in einem natürlich vorkommenden oder synthetischen Polymer, und
b) die Omega-3-Fettsäuren Eicosapentaensäure (EPA) und Docosahexaensäure (DHA) in Form von entweder Fettsäuresalzen mit einem organischen Gegenion, ausgewählt aus Lysin, Arginin, Ornithin, Cholin und Mischungen desselben, oder
Mischungen aus freien Fettsäuren und Omega-3-Fettsäuresalzen mit einem organischen Gegenion, ausgewählt aus Lysin, Arginin, Ornithin, Cholin:
wobei Komponente a) und b) eine Mischung in der Zubereitung bilden.

2. Zubereitung nach Anspruch 1, wobei L-Rhamnose entweder in dessen monomerer Form oder enthalten in einem natürlich vorkommenden oder synthetischen Polymer vorliegt, ausgewählt aus Pektinen, Hemicellulose, Gummi arabicum, Karaya-Gummi, bakteriellen Polysacchariden, Ulvan, Rhamnolipiden und Glykosiden Naringin, Hesperidin oder Quercetrin.

3. Zubereitung nach Anspruch 2, wobei es sich bei dem Polymer um Pektin handelt, vorzugsweise aus Äpfeln oder Zitrusfrüchten.

4. Zubereitung nach einem der vorhergehenden Ansprüche, wobei EPA und DHA in der Form von Fettsäuresalzen mit einem organischen Gegenion vorliegen, das aus Lysin, Arginin, Ornithin, Cholin und Mischungen desselben, vorzugsweise aus Lysin, Arginin und Ornithin ausgewählt ist.

5. Zubereitung nach einem der vorhergehenden Ansprüche, ferner umfassend eines oder mehrere des Folgenden: probiotische Stämme, vorzugsweise ausgewählt aus Propionibakterium, *Veillonella alcalescens, Clostridium propionicum, Selenomonas, Micromonospora, Bacteroides* und *Ruminococcus, Bacillus,* vorzugsweise ausgewählt aus *B. subtilis, B. licheniformis* oder *B. amyloliquefaciens,* Präbiotika, Aminosäuren, Aminosäuresalzen, Peptiden.

6. Zubereitung nach einem der vorhergehenden Ansprüche, ferner umfassend eines oder mehrere des Folgenden: Pektin, Rhamnogalacturonan, Laminarin, Galactomannan, Gersten-β-Glucan, Pyrodextrin, Pullulan, Arabinoxylan, Inulin, Fructooligosaccharide, Galactooligosaccharide.

7. Zubereitung nach einem der vorhergehenden Ansprüche, umfassend mindestens 10 Gew.-% Omega-3-Fettsäure, vorzugsweise mindestens 20 Gew.-%, noch bevorzugter mindestens 30 Gew.-%, am meisten bevorzugt mindestens 40 Gew.-% Omega-3-Fettsäure.

8. Zubereitung nach einem der vorhergehenden Ansprüche, umfassend mindestens 10 Gew.-% L-Rhamnose, vorzugsweise mindestens 20 Gew.-%, noch bevorzugter mindestens 30 Gew.-%, am meisten bevorzugt mindestens 40 Gew.-% L-Rhamnose.

9. Pharmazeutische oder nutrazeutische Darreichungsform, umfassend eine Zubereitung nach einem der vorhergehenden Ansprüche, wobei es sich bei der Darreichungsform um eine Tablette oder Kapsel handelt und das Gesamtgewicht von L-Rhamnose, EPA und DHA nicht mehr als 5 g, vorzugsweise nicht mehr als 3 g, noch bevorzugter nicht mehr als 1 g beträgt.

10. Pharmazeutische oder nutrazeutische Darreichungsform, umfassend eine Zubereitung nach einem der Ansprüche 1 bis 8, wobei es sich bei der Darreichungsform um Granulat, Streusel oder Sachets handelt und das Gesamtgewicht von L-Rhamnose, EPA und DHA nicht mehr als 50 g, vorzugsweise nicht mehr als 30 g, noch bevorzugter nicht mehr als 10 g beträgt.

11. Pharmazeutische oder nutrazeutische Darreichungsform nach Anspruch 9 oder 10, ferner umfassend ein Kolon-spezifisches Freisetzungssystem, umfassend vorzugsweise eine Beschichtung, die vorzugsweise ausgewählt ist aus Methylacrylat-Methacrylsäure-Copolymeren, Celluloseacetatphthalat (CAP), Celluloseacetatsuccinat, Hydroxypropylmethylcellulosephthalat, Hydroxypropylmethylcelluloseacetatsuccinat (Hypromelloseacetatsuccinat), Polyvinylacetatphthalat (PVAP), Methylmethacrylat-Methacrylsäure-Copolymeren, Schellack, Celluloseacetattrimellitat, Natriumalginat, Zein.

12. Pharmazeutische oder nutrazeutische Darreichungsform nach Anspruch 11, wobei die Beschichtung Pektin oder ein Pektinsalz, vorzugsweise Calciumpectinat, umfasst.

13. Futter- oder Lebensmittelzusammensetzung, enthaltend eine Zubereitung nach einem der Ansprüche 1 bis 8 und mindestens einen weiteren Futter- oder Lebensmittelbestandteil, der vorzugsweise aus Proteinen, Kohlenhydraten, Fetten, weiteren Probiotika, Präbiotika, Enzymen, Vitaminen, Immunmodulatoren, Milchaustauschstoffen, Mineralien, Aminosäuren, Kokzidiostatika, Produkten auf Säurebasis, Arzneimitteln und Kombinationen davon ausgewählt ist.

14. Zubereitung nach einem der Ansprüche 1 bis 8 zur Verwendung als Futter- oder Nahrungsergänzungsmittel oder als pharmazeutische Zusammensetzung.

15. Zubereitung nach einem der Ansprüche 1 bis 8 zur Verwendung als Medikament, vorzugsweise für: kardiovaskuläre Gesundheit, metabolische Gesundheit, Glukose- und Fettstoffwechsel, Insulinsensitivität, Lebergesundheit, Gewichtsmanagement/Gewichtsabnahme/- kontrolle, neuronale Gesundheit.

16. Zubereitung nach einem der Ansprüche 1 bis 8 zur Verwendung bei der Behandlung oder Vorbeugung einer Erkrankung oder Störung, ausgewählt aus Hyperlipidämie, Hypercholesterinämie, nichtalkoholischer Fettleber, Hepatitis, Typ-2-Diabetes, Prädiabetes, Glukoseintoleranz, Arteriosklerose, anderen Gefäßerkrankungen, Fettleibigkeit, Adipositas, Multipler Sklerose.

## Revendications

1. Préparation comprenant
a) du L-rhamnose soit sous sa forme monomérique, soit contenu dans un polymère d'origine naturelle ou synthétique, et
b) les acides gras oméga-3 acide eicosapentaénoïque (EPA) et acide docosahexaénoïque (DHA) sous la forme soit de sels d'acides gras ayant un contre-ion organique choisi parmi la lysine, l'arginine, l'ornithine, la choline et leurs mélanges, soit
des mélanges d'acides gras libres et de sels d'acides gras oméga-3 ayant un contre-ion organique choisi parmi la lysine, l'arginine, l'ornithine, la choline :
les composants a) et b) formant un mélange dans la préparation.

2. Préparation selon la revendication 1, dans laquelle le L-rhamnose est soit sous sa forme monomérique, soit contenu dans un polymère d'origine naturelle ou synthétique choisi parmi les pectines, l'hémicellulose, la gomme arabique, la gomme karaya, les polysaccharides bactériens, l'ulvane, les rhamnolipides, et les glycosides, la naringine, l'hespéridine, ou la quercitrine.

3. Préparation selon la revendication 2, dans laquelle le polymère est une pectine, de préférence de pommes ou d'agrumes.

4. Préparation selon l'une quelconque des revendications précédentes, dans laquelle l'EPA et le DHA sont sous forme de sels d'acides gras ayant un contre ion organique choisi parmi la lysine, l'arginine, l'ornithine, la choline et leurs mélanges, de préférence choisi parmi la lysine, l'arginine et l'ornithine.

5. Préparation selon l'une quelconque des revendications précédentes, comprenant en outre une ou plusieurs des suivants : souches probiotiques, de préférence choisies parmi Propionibacterium, Veillonella alcalescens, Clostridium propionicum, Selenomonas, Micromonospora, Bacteroides, et Ruminococcus, Bacillus, de préférence choisies parmi B. subtilis, B. licheniformis ou B. amyloliquefaciens, prébiotiques, acides aminés, sels d'acides aminés, peptides.

6. Préparation selon l'une quelconque des revendications précédentes, comprenant en outre un ou plusieurs des suivants : pectine, rhamnogalacturonane, laminarine, galactomannane, β-glucane d'orge, pyrodextrine, pullulane, arabinoxylane, inuline, fructo-oligosaccharides, galacto-oligosaccharides.

7. Préparation selon l'une quelconque des revendications précédentes, comprenant au moins 10 % en poids d'acide gras oméga-3, de préférence au moins 20 % en poids, plus préférablement au moins 30 % en poids, le plus préférablement au moins 40 % en poids d'acide gras oméga-3.

8. Préparation selon l'une quelconque des revendications précédentes comprenant au moins 10 % en poids de L-rhamnose, de préférence au moins 20 % en poids, plus préférablement au moins 30 % en poids, le plus préférablement au moins 40 % en poids de L-rhamnose.

9. Forme posologique pharmaceutique ou nutraceutique comprenant une préparation selon l'une quelconque des revendications précédentes, dans laquelle la forme posologique est un comprimé ou une capsule et le poids total de L-rhamnose, EPA et DHA n'est pas supérieur à 5 g, de préférence pas supérieur à 3 g, plus préférablement pas supérieur à 1 g.

10. Forme posologique pharmaceutique ou nutraceutique comprenant une préparation selon l'une quelconque des revendications 1 à 8, dans laquelle la forme posologique comprend des granules, des saupoudrés ou des sachets et le poids total de L-rhamnose, EPA et DHA n'est pas supérieur à 50 g, de préférence pas supérieur à 30 g, plus préférablement pas supérieur à 10 g.

11. Forme posologique pharmaceutique ou nutraceutique selon la revendication 9 ou 10, comprenant en outre un système d'administration spécifique au côlon, comprenant de préférence un revêtement, choisi de préférence parmi les copolymères d'acrylate de méthyle-acide méthacrylique, acétate phtalate de cellulose (CAP), succinate acétate de cellulose, phtalate d'hydroxypropylméthylcellulose, succinate acétate d'hydroxypropylméthylcellulose (succinate acétate d'hypromellose), phtalate acétate de polyvinyle (PVAP), copolymères de méthacrylate de méthyle et d'acide méthacrylique, gomme laque, trimellitate acétate de cellulose, alginate de sodium, zéine.

12. Forme posologique pharmaceutique ou nutraceutique selon la revendication 11, dans laquelle le revêtement comprend une pectine ou un sel de pectine, de préférence un pectinate de calcium.

13. Composition alimentaire ou alimentaire pour animaux contenant une préparation selon l'une quelconque des revendications 1 à 8 et au moins un autre ingrédient alimentaire ou alimentaire pour animaux, de préférence choisi parmi les protéines, les glucides, les graisses, d'autres probiotiques, les prébiotiques, les enzymes, les vitamines, les modulateurs immunitaires, les substituts du lait, les minéraux, les acides aminés, les coccidiostatiques, les produits à base d'acides, les médicaments et des combinaisons de ceux-ci.

14. Préparation selon l'une quelconque des revendications 1 à 8 pour une utilisation comme supplément pour produit alimentaire pour animaux ou pour produit alimentaire ou dans une composition pharmaceutique.

15. Préparation selon l'une quelconque des revendications 1 à 8, pour une utilisation comme médicament, de préférence pour : la santé cardiovasculaire, la santé métabolique, le métabolisme du glucose et des lipides, la sensibilité à l'insuline, la santé du foie, la gestion du poids / perte / régulation du poids, la santé neuronale.

16. Préparation selon l'une quelconque des revendications 1 à 8, pour une utilisation dans le traitement ou la prévention d'une maladie ou d'un trouble choisi(e) parmi l'hyperlipidémie, l'hypercholestérolémie, le foie gras non alcoolique, l'hépatite, le diabète de type 2, prédiabète, l'intolérance au glucose, l'artériosclérose, d'autres maladies vasculaires, l'obésité, l'adipose, la sclérose en plaques.
